# FASCICULE DE BREVET EUROPEEN

(11) **EP 0 966 308 B1**
(45) Date de publication et mention de la délivrance du brevet: **08.05.2002**
(21) Numéro de dépôt: 98912567.9
(22) Date de dépôt: 27.02.1998
(51) Int. Cl.: A61L 9/03

(54) **DIFFUSEUR DE PARFUM**
PARFÜM-SPENDER
PERFUME DIFFUSER

(30) Priorité: 28.02.1997 FR 9702451
(43) Date de publication de la demande: 29.12.1999
(73) Titulaire: PRODUITS BERGER S.A., 75001 Paris (FR)
(72) Inventeur: LEHOUX, Jannick, F-27370 Thuit-Signol (FR); GOMEZ, Corinne, F-76410 Cleon (FR)
(74) Mandataire: Somnier, Jean-Louis
(86) Numéro de dépôt international: FR9800391
(87) Numéro de publication internationale: WO9837922

(56) Documents cités:
- WO-A-94/04200
- DE-A- 3 327 152
- US-A- 5 556 192
- US-A- 5 563 981

## Description

La présente invention concerne un diffuseur de parfum, et un système pour diffuser un parfum comportant un diffuseur de parfum selon la présente invention.

Dans ce qui suit, la diffusion d'un parfum couvre à la fois l'évaporation d'un parfum ou d'une matière parfumée à l'état liquide ou de gel et la sublimation d'un parfum ou d'une matière parfumée à l'état solide.

On connaît le brûle-parfum, qui est un évaporateur de parfum adapté à coopérer avec une source de chaleur et avec des moyens adaptés à recevoir ou à constituer un récipient contenant une matière adaptée à s'évaporer ou à se sublimer en diffusant un parfum, le diffuseur et ces moyens étant agencés de manière à transmettre à ladite matière une partie de la chaleur produite par ladite source.

Selon une configuration classique du brûle-parfum, la source de chaleur est une petite bougie qui est placée sous le récipient contenant en général un liquide adapté à l'évaporation d'un parfum, et qui chauffe ce récipient pour chauffer ladite matière afin que celle-ci s'évapore.

Ce brûle-parfum présente plusieurs inconvénients. Il est impossible de régler la température de la matière adaptée à diffuser un parfum, en cours d'utilisation du brûle-parfum, à cause des variations de la hauteur entre la flamme et le parfum. De façon plus particulière, il est très difficile de maintenir la température de cette matière dans la plage de température allant de 40 à 65°C environ qui est la plage de température idéale pour la diffusion d'un parfum. Le réglage difficile de la température de la matière rend difficile le réglage de la vitesse de diffusion du parfum. La température atteinte en cours d'évaporation de la matière parfumée se situe au-delà de 65°C, ce qui limite le choix des parfums et réduit leur qualité olfactive. La capacité du réservoir contenant la matière parfumée est limitée, et oblige l'utilisateur à le recharger fréquemment pour obtenir un fonctionnement continu de plusieurs heures (durée de vie d'une bougie par exemple).

L'ensemble du brûle-parfum est chauffé, et peut provoquer des sensations de brûlures.

L'endroit où est posé la bougie n'est pas toujours facile d'accès.

En outre, étant donné que la bougie doit être placée sous le récipient contenant la matière, la flamme de la bougie est peu visible et est donc difficilement utilisable pour créer une ambiance chaleureuse et feutrée comme une bougie ordinaire.

Le but de la présente invention est de remédier aux inconvénients des diffuseurs de parfum connus, et de proposer un diffuseur de parfum du type précité qui soit simple et économique, qui permette d'obtenir une vitesse de diffusion du parfum relativement régulière, et qui, dans le cas de l'utilisation d'une flamme comme source de chaleur, permette de rendre cette flamme bien visible.

Ce diffuseur de parfum doit en outre pouvoir être intégré dans des systèmes pour diffuser un parfum de tous types et de tous styles sans risques de brûlures.

Suivant l'invention, le diffuseur de parfum du type précité est caractérisé en ce qu'il comporte :
- un premier élément conducteur de la chaleur adapté à recevoir une partie de la chaleur produite par ladite source de chaleur, et à transmettre par conduction thermique la chaleur reçue ;
- un second élément conducteur de la chaleur séparé du premier élément et adapté à être en contact avec ladite matière;
- un troisième élément conducteur de la chaleur relié au premier élément conducteur et au second élément conducteur de manière à communiquer par conduction thermique au second élément conducteur la chaleur transmise par le premier élément conducteur.
- l'un ou l'autre ou chacun des premier et troisième éléments conducteurs étant le cas échéant isolé thermiquement sur une partie au moins de sa surface extérieure.

La présence des trois éléments conducteurs de la chaleur permet de régler séparément la chaleur reçue par le premier élément conducteur, la chaleur transmise du premier élément conducteur au troisième élément conducteur, enfin la chaleur transmise par ce troisième élément conducteur à la matière adaptée à diffuser un parfum.

On peut ainsi définir ces trois éléments conducteurs de la chaleur de manière permettant d'obtenir une température sensiblement constante de la matière adaptée à diffuser le parfum.

Dans le cas où la source de chaleur est une flamme, le diffuseur de parfum permet de disposer cette flamme d'une manière telle qu'elle soit visible.

Enfin, la présence des trois éléments conducteurs de la chaleur ne limite en rien les types et les styles du diffuseur de parfum ou du système pour diffuser un parfum comportant un tel diffuseur.

Suivant une version avantageuse de l'invention, les éléments conducteurs de la chaleur sont agencés et dimensionnés de manière à transmettre par conduction thermique à la matière un flux de chaleur suffisant pour chauffer ladite matière à une température sensiblement constante comprise entre 40°C et 65°C environ.

On peut ainsi obtenir une vitesse de diffusion et une intensité du parfum sensiblement constantes et d'une valeur correspondant aux souhaits des utilisateurs.

Un tel diffuseur de parfum permet de parfumer l'ambiance par sublimation ou évaporation d'un parfum ou d'une matière parfumée (liquide, solide, ou gel) à l'aide d'un système conducteur thermique faisant partie intégrante du parfum ou de la matière parfumée. Ce système thermique, pour fonctionner, nécessite l'utilisation d'une flamme ou d'autres sources de chaleur, et par conduction thermique, communique une quantité d'énergie prédéterminée à la matière parfumée suffisante pour que celle-ci s'évapore ou se sublime, avec une vitesse de diffusion relativement régulière. La capacité du réservoir est de préférence suffisamment importante pour permettre un fonctionnement en continu de plusieurs heures (exemple : durée de vie d'une bougie), sans obliger l'utilisateur à le remplir souvent. La plage de température utilisée permet de choisir les parfums parmi un grand éventail de matières odorantes, y compris les plus légères qui s'évaporent aux températures les plus basses à partir de 40°C, sans aucune limitation créée par une maîtrise insuffisante de la température de la matière à diffuser.

Suivant une version préférée de l'invention, le troisième élément conducteur présente, au moins dans le tronçon dudit élément qui est en contact avec la matière parfumée, une dimension transversale qui s'évase dans le sens vers le second élément.

Au fur et à mesure de l'élimination de la matière adaptée à diffuser le parfum, la longueur du tronçon dudit élément qui est en contact avec la matière diminue, tandis que la largeur de ce tronçon augmente. Ceci permet de maintenir un rapport sensiblement constant entre la surface d'échange thermique entre les second et troisième éléments conducteurs de la chaleur d'une part, et le volume de la matière adaptée à diffuser le parfum, de façon à maintenir un apport calorifique suffisant pour maintenir une température sensiblement constante de ladite matière et une vitesse sensiblement constante de diffusion du parfum.

Suivant un autre aspect de l'invention, le système pour diffuser un parfum selon l'invention, comportant une source de chaleur et un récipient contenant une matière adaptée à s'évaporer ou à se sublimer en diffusant un parfum, est caractérisé en ce qu'il comporte un diffuseur de parfum selon la première version de la présente invention.

D'autres particularités et avantages de la présente invention apparaîtront dans la description détaillée ci-après.

Aux dessins annexés, donnés uniquement à titre d'exemple non limitatifs :
- la figure 1 est une vue en élévation schématisant la structure d'un système pour diffuser un parfum selon le second aspect de l'invention, comprenant un diffuseur de parfum selon un mode de réalisation du premier aspect de la présente invention ;
- la figure 2 est une vue en élévation, en coupe selon II-II à la figure 4, du diffuseur de parfum selon le mode de réalisation de la figure 1 ;
- la figure 3 est une vue du diffuseur de parfum de la figure 2 depuis la gauche de cette figure ;
- la figure 4 est une vue de dessus du diffuseur de parfum de la figure 2 ;
- la figure 5 est un schéma représentant deux variantes du système de la figure 1 ;
- la figure 6 est un diagramme montrant l'évolution de la diffusion d'un parfum dans le temps avec un système pour diffuser un parfum ou une matière parfumée et un diffuseur de parfum selon la présente invention.

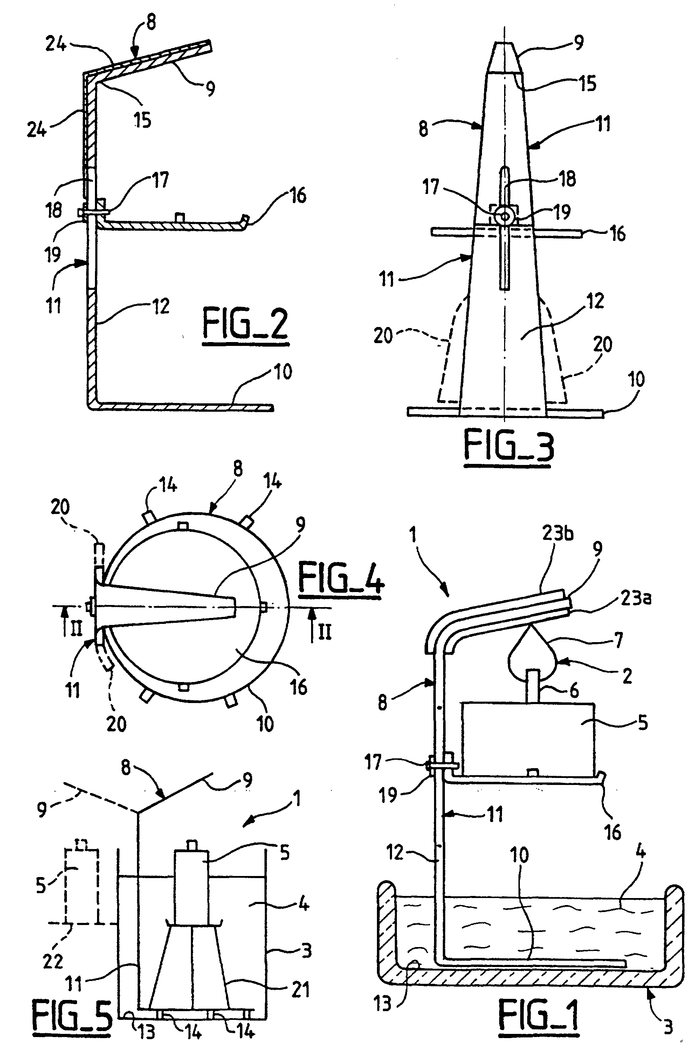

Dans le mode de réalisation représenté schématiquement à la figure 1, le système 1 pour diffuser un parfum ou une matière parfumée, comporte une source 2 de chaleur et un récipient 3 contenant une matière 4 adaptée à s'évaporer ou à se sublimer en diffusant un parfum.

De façon classique, la source 2 de chaleur est, par exemple, une petite bougie comprenant un produit combustible 5 et une mèche 6 adaptée à s'imprégner de produit combustible 5. Lorsque la bougie 2 est allumée, elle produit une flamme schématisée en 7 qui dégage de la chaleur.

Le récipient 3 est un récipient de forme quelconque réalisé en un matériau quelconque et adapté à contenir la matière 4 et à supporter la température à laquelle cette matière est portée.

La matière 4 adaptée à s'évaporer ou à se sublimer en diffusant un parfum est une matière quelconque connue, à l'état liquide, solide ou à l'état de gel. Pour supprimer tout risque d'incendie, la matière 4 est habituellement une matière aqueuse sous forme de liquide, de solide ou de gel, contenant une substance parfumée adaptée à diffuser lorsque la matière 4 s'évapore ou se sublime.

Le système 1 comporte un diffuseur de parfum 8 selon un mode de réalisation de l'invention représenté en détail aux figures 2 à 4.

Dans le mode de réalisation des figures 2 à 4, le diffuseur de parfum 8 est adapté à coopérer avec une source 2 de chaleur et avec des moyens adaptés à recevoir ou à constituer un récipient 3 contenant une matière 4 adaptée à s'évaporer ou à se sublimer en diffusant un parfum. Le diffuseur 8 et ces moyens sont agencés de manière à transmettre à la matière 4 une partie de la chaleur produite par la source 2 de chaleur.

Suivant la présente invention, le diffuseur de parfum 8 comporte :
- un premier élément 9 conducteur de la chaleur, isolé thermiquement ou non sur sa face externe, adapté à recevoir une partie de la chaleur produite par la source 2 de chaleur et à transmettre par conduction thermique la chaleur reçue ;
- un second élément 10 conducteur de la chaleur séparé du premier élément 9 et adapté à être en contact avec la matière 4 ;
- un troisième élément 11 conducteur de la chaleur, isolé thermiquement ou non sur sa face externe depuis la languette 9 sensiblement sur toute la hauteur qui reste à l'air libre (voir plus loin), relié au premier élément conducteur 9 et au second élément conducteur 10 de manière à communiquer par conduction thermique au second élément conducteur 10 une partie au moins de la chaleur transmise par le premier élément conducteur 9.

Les éléments 9, 10, 11 conducteurs de la chaleur, sont agencés et dimensionnés de manière à transmettre par conduction thermique à la matière 4 un flux de chaleur suffisant pour chauffer ladite matière 4 à une température sensiblement constante comprise en 40°C et 65°C environ.

On sait que la plage de température allant de 40°C à 65°C environ est la plage idéale pour diffuser un parfum.

Comme représenté aux figures, les trois éléments 9, 10, 11 conducteurs de la chaleur sont réalisés d'une seule pièce, le diffuseur de parfum 8 étant réalisé principalement en métal bon conducteur de la chaleur et résistant à la température créée par la source 2, par exemple en aluminium ou en cuivre, ou en tout autre métal adéquat. D'autres modes de réalisation sont possibles.

Dans l'exemple représenté, le diffuseur de parfum 8 est réalisé en tôle découpée et pliée, par exemple en une tôle d'aluminium ou de cuivre d'épaisseur voisine de 1 mm environ. Le diffuseur 8 pourrait être réalisé par moulage ou par formage à la presse.

Dans l'exemple représenté, le diffuseur de parfum 8 est adapté à coopérer avec une source 2 de chaleur constituée par une flamme 7, par exemple la flamme 7 d'une bougie.

Le premier élément 9 conducteur de la chaleur est une languette 9 de métal inclinée par rapport à la direction de la flamme 7, c'est-à-dire la direction verticale, et touchant ou non ladite flamme 7 à l'extrémité de celle-ci lors du fonctionnement du diffuseur 8. La languette 9 est ainsi située à la partie supérieure du diffuseur 8.

La languette 9 est réalisée de manière telle que son inclinaison par rapport à la direction de la flamme 7 est de préférence réglable (voir plus loin).

D'une manière plus générale, le diffuseur de parfum 8 comporte des moyens, qui seront détaillés ci-après, pour modifier la distance entre la languette 9 et la flamme 7, afin de régler la vitesse de diffusion du parfum.

Le second élément 10 conducteur de la chaleur est adapté à être noyé dans la matière 4, et le troisième élément conducteur 11 comporte un tronçon 12 adjacent au second élément conducteur 10 et adapté à être également noyé dans la matière 4.

Le second élément 10 conducteur de la chaleur s'étend sensiblement sur toute la surface du fond 13 du récipient 3 contenant la matière 4 (voir figure 1).

Le second élément 10 conducteur de la chaleur repose sur le fond 13 du récipient 3 contenant la matière 4, soit directement, soit par des entretoises 14 le maintenant à distance dudit fond 13, ce qui augmente la surface d'échange thermique entre le second élément conducteur 10 et la matière 4.

Dans l'exemple représenté aux figures 2 à 4, le second élément 10 a une forme sensiblement circulaire qui s'inscrit facilement dans la section transversale du récipient 3 de forme quelconque. Il est situé à la partie inférieure du diffuseur 8.

Le troisième élément conducteur 11 présente, au moins dans le tronçon 12 dudit élément 11 qui est susceptible d'être en contact avec la matière 4, une dimension transversale qui s'évase dans le sens vers le second élément 11.

Le troisième élément conducteur 11 a, dans l'exemple représenté, une fonction de montant pour supporter, d'une part, la languette 9 qui est reliée au montant 11 par le pli 15, d'autre part la coupelle 16 adaptée à supporter la bougie 2 ou une autre source de chaleur placée sous la languette 9.

La coupelle 16 est fixée de manière réglable au montant 11 par l'intermédiaire d'un boulon 17 traversant une lumière longitudinale verticale 18 du montant 11, une rondelle 19 étant introduite entre la tête du boulon 17 opposée à la coupelle 16, et le montant 11.

D'après les premiers essais de la Demanderesse, il semble très important que le montant 11 ait, au moins dans son tronçon 12 adjacent au second élément conducteur 10, une dimension transversale qui s'évase dans le sens vers le second élément 10. Ceci a pour effet d'augmenter le rapport entre la surface totale d'échange thermique entre le diffuseur 8 et la matière 4 d'une part, et la hauteur résiduelle de la matière 4 dans le récipient 3, et donc le volume résiduel de la matière 4 dans ledit récipient 3, d'autre part. Ceci permet d'obtenir une vitesse de diffusion du parfum sensiblement constante dans le temps malgré la disparition progressive de la matière 4 et la diminution du niveau et du volume de cette matière.

Pour cette raison, le montant 11 peut avoir une forme trapézoïdale régulière, comme représenté à la figure 3. Ce montant 11 peut également présenter, dans sa partie inférieure 12 adaptée à plonger dans la matière 4, des ailes latérales 20, schématisées en tirets aux figures 3 et 4, qui peuvent avoir une forme et une orientation quelconques, et peuvent s'étendre par exemple soit dans le plan du montant 11, soit selon une surface courbe schématisée dans la partie inférieure de la figure 4.

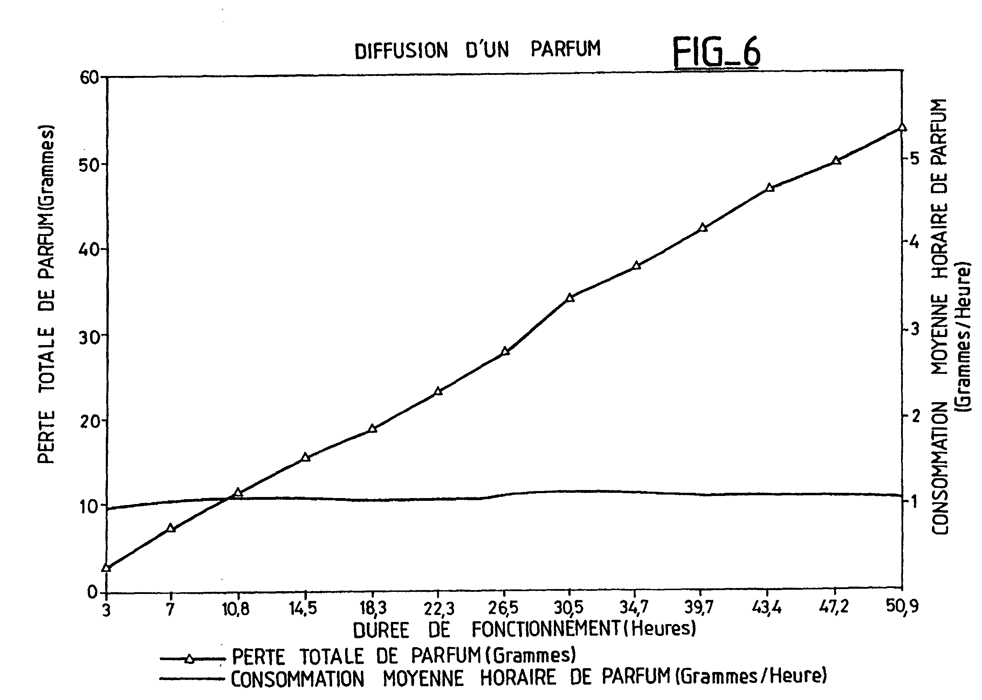

Dans la réalisation de la figure 5, on a représenté en traits pleins une bougie 2 posée sur un trépied 21 reposant directement sur le second élément 10 : la bougie 2 est donc située au-dessus du second élément 10 et au-dessous de la languette 9, sans être supportée par le montant 11. On a également représenté en tirets à cette figure une variante dans laquelle la bougie 2 est supportée par un support 22 en porte à faux par rapport au récipient 3, la languette 9 étant pliée vers l'extérieur du récipient pour être située au-dessus du support 22.

Pour régler la distance entre la languette 9 et la source de chaleur 2, 7, pour suivre par exemple la diminution de la hauteur d'une bougie 5 au fur et à mesure du fonctionnement de celle-ci, on peut modifier l'angle de la languette 9 par rapport au montant 11, par pliage de la languette 9 autour du pli 15 vers la flamme 7. Un pliage en sens inverse intervient en cas de remplacement d'une bougie usée par une neuve.

On peut également prévoir avec la languette 9 des moyens entraînant automatiquement un pliage de celle-ci vers la flamme 7 lorsque la température de la languette 9 diminue. On peut ainsi associer à la languette 9 une languette auxiliaire 23 soudée à ses extrémités à la languette 9 de manière à constituer un bilame, la position et la nature du métal de la languette 23 étant choisies de manière à atteindre le résultat recherché, un tel dispositif fonctionnant dans les deux sens.

Ainsi, comme schématisé à la figure 1, la languette 23a peut être placée sous la languette 9 si elle est réalisée en un métal qui se rétracte plus vite que le métal de la languette 9 en cas de baisse de la température. Au contraire, une languette 23b en un métal se rétractant moins vite que celui de la languette 9 sera située au-dessus de cette dernière.

En variante, c'est le montant 11 qui peut être conçu avec une structure de bilame pour déplacer la languette 9 par rapport à la flamme 7.

Pour limiter les pertes de chaleur par rayonnement du diffuseur 8, et pour communiquer à la base 10 et à la matière parfumée 4 une proportion maximale de la quantité de la chaleur reçue par la languette 9, la languette 9 et le montant 11 sont isolés thermiquement sur une partie au moins de sa surface extérieure.

Dans l'exemple représenté, la languette 9 est revêtue, sur sa surface externe opposée à la flamme 7, d'un revêtement 24 thermiquement isolant.

De même, le montant 11 est revêtu, au moins sur sa face externe, d'un revêtement isolant 24, sensiblement au-dessus du niveau de la coupelle 16. De cette manière, la partie inférieure 12 susceptible d'entrer en contact avec la matière parfumée 4 reste nue pour permettre un échange thermique optimal avec cette dernière.

Bien entendu, le revêtement 24 peut être conçu également comme revêtement décoratif, ou recouvert d'un revêtement décoratif quelconque.

On a ainsi décrit un diffuseur de parfum de structure extrêmement simple, et donc très économique, qui se prête à tous les modes de réalisation possibles ; dans le cas où la source de chaleur est une flamme, par exemple la flamme d'une bougie, le diffuseur de parfum 8 permet de placer la flamme en évidence pour que la flamme participe à la création d'une atmosphère chaleureuse, notamment la nuit. Ce diffuseur reçoit par la languette 9 de l'énergie calorifique de la flamme, et l'énergie transmise à la matière parfumée 4 permet d'atteindre une température nettement inférieure à celle de la flamme, située dans la plage recherchée de 40°C à 65°C environ.

Le diffuseur de parfum selon l'invention permet, de façon tout à fait surprenante, d'obtenir une vitesse de diffusion par évaporation d'un parfum relativement constante dans le temps. Ceci se voit à l'examen de la figure 6 qui représente une courbe d'évolution de la diffusion d'un parfum dans le temps au cours d'un essai effectué dans les conditions ci-dessous. Cet essai a duré environ 51 heures et a donné les résultats ci-dessous :

| Durée d'utilisation du système (h) | perte totale de parfum en poids (g) | consommation horaire moyenne de parfum (g/h) |
|---|---|---|
| 3 | 2,84 | 0,95 |
| 7 | 7,3 | 1,04 |
| 10,8 | 11,31 | 1,05 |
| 14,5 | 15,35 | 1,06 |
| 18,3 | 18,76 | 1,02 |
| 22,3 | 23,1 | 1,04 |
| 26,5 | 27,72 | 1,05 |
| 30,5 | 34,01 | 1,12 |
| 34,7 | 37,61 | 1,09 |
| 39,7 | 42,06 | 1,06 |
| 43,4 | 46,59 | 1,07 |
| 47,2 | 49,7 | 1,05 |
| 50,9 | 53,5 | 1,05 |

Les essais ont été réalisés avec un système selon les figures 1 à 4 et un diffuseur d'environ 8 cm de hauteur, la source de chaleur étant une bougie.

La perte totale de parfum est le poids total perdu à un instant t depuis le début de l'essai, et correspond à l'échelle de gauche à la figure 6.

La consommation horaire moyenne est la perte totale de parfum à un instant t divisée par le nombre des heures écoulées depuis le début de l'essai, et correspond à l'échelle de droite à la figure 6.

Bien entendu, la présente invention n'est pas limitée aux modes de réalisation que l'on vient de décrire, et on peut apporter à ceux-ci de nombreux changements et modifications sans sortir du domaine de l'invention.

On peut en particulier remplacer la flamme décrite par toute autre source de chaleur. On peut ainsi concentrer au moyen d'une loupe les rayons du soleil pour chauffer la languette conductrice 9, ou utiliser une source de rayonnement infrarouge à cet effet. La source de chaleur 2 peut être placée dans une position quelconque dans l'espace par rapport à la matière 4.

Bien entendu, la quantité de chaleur transmise par conduction à la matière parfumée n'est pas strictement égale à la quantité de chaleur reçue par le premier élément conducteur, des pertes intervenant par conduction, rayonnement et convection à partir des divers éléments conducteurs. Ces pertes peuvent varier en fonction des paramètres concernant l'atmosphère dans laquelle est installé le diffuseur, la température, l'humidité, le degré d'agitation de l'air ambiant. Ces pertes varient également lorsque varie la surface de contact entre les éléments conducteurs et la matière parfumée, au fur et à mesure de la consommation de ladite matière.

On peut bien entendu placer la source de chaleur sur un support indépendant des éléments conducteurs, prévoir plusieurs sources de chaleur, associées à un seul ou à plusieurs ensembles d'éléments conducteurs.

On peut donner au second élément conducteur adapté à être en contact avec la matière parfumée toute forme compatible avec la fonction de cet élément. On peut en particulier faire en sorte que les deux ailes 20 aient sensiblement la forme de deux demi-cylindres de même axe et d'un diamètre prédéterminé de façon telle que ces ailes prennent appui élastiquement contre les parois du récipient contenant la matière parfumée tout en constituant une surface importante d'échange thermique avec ladite matière, en association le cas échéant avec un élément de fond 10, le fond du récipient pouvant avoir une forme quelconque non plane.

Les éléments conducteurs brûlants peuvent avantageusement être protégés par un matériau isolant, notamment du genre céramique poreuse ; ils peuvent être par exemple noyés dans un tel matériau ou entourés d'une gaine en un tel matériau.

L'ensemble constitué par les premier, second et troisième éléments conducteurs peut avoir, en élévation, une forme générale, plus ou moins stylisée, de chiffre, par exemple 2, 3, 5, 6, 8, 9, ou de lettre, par exemple A, B, C, E, G, O ou θ, P, R, S, Z, d, b, a. La source de chaleur peut si on le désire être déposée sur une partie horizontale appartenant à cette forme ou ajoutée à celle-ci. En particulier, la forme Z offre, par la barre transversale oblique, une surface d'échange qui diminue relativement peu lorsque la hauteur de matière parfumée diminue.

La plupart des formes évoquées ci-dessus peuvent être obtenues par découpage et cintrage d'une tôle.

On peut également procéder par formage et emboutissage d'une tôle à la presse. On peut ainsi, en variante par rapport aux deux ailes semi-cylindriques décrites ci-dessus, concevoir le second élément conducteur comme un récipient, de section sensiblement cylindrique, adapté à contenir directement la matière parfumée, et fabriqué au moyen d'une presse, le premier et le troisième éléments conducteurs selon la présente invention étant associés d'une manière quelconque à ce récipient, en ne formant de préférence qu'une seule pièce avec celui-ci.

Le récipient ainsi obtenu, qui remplit à lui seul toutes les fonctions techniques du diffuseur de parfum selon la présente invention, est adapté à être introduit dans une enceinte ou un conteneur de forme quelconque qui peut être conçu(e) en fonction de critères essentiellement économiques et esthétiques.

On peut également réaliser le diffuseur par moulage de tout matériau approprié.

Le diffuseur selon l'invention peut aussi être réalisé en des matériaux autres que des métaux bons conducteurs de la chaleur, par exemple en verre ou tout autre matériau, notamment fondu, résistant à la température de la flamme ou de la source de chaleur. Les divers éléments conducteurs formant le diffuseur sont alors dimensionnés de manière à remplir les fonctions décrites ci-dessus pour le diffuseur, et en particulier à communiquer à la matière parfumée la quantité de chaleur nécessaire pour la porter à la température requise.

## Revendications

1. Diffuseur de parfum (8), agencé pour coopérer avec une source (2) de chaleur et avec des moyens agencés pour recevoir ou constituer un récipient (3) contenant une matière parfumée (4) adaptée à s'évaporer ou à se sublimer en diffusant un parfum, le diffuseur (8) et ces moyens étant agencés de manière à transmettre à ladite matière parfumée (4) une partie de la chaleur produite par ladite source (2), **caractérisé en ce qu'**il comporte :
- un premier élément (9) conducteur de la chaleur séparé et situé à distance du récipient (3) et agencé pour recevoir une partie de la chaleur produite par ladite source (2) de chaleur, et pour transmettre par conduction thermique une partie de la chaleur reçue ;
- un second élément (10) conducteur de la chaleur séparé et situé à distance du premier élément (9) et agencé pour être en contact avec ladite matière (4) ;
- un troisième élément (11) conducteur de la chaleur relié au premier élément conducteur (9) et au second élément conducteur (10) de manière à communiquer par conduction thermique au second élément conducteur (10) une partie de la chaleur transmise par le premier élément conducteur (9) ;
- l'un ou l'autre ou chacun des premier et troisième éléments conducteurs (9, 11) étant le cas échéant isolé thermiquement sur une partie au moins de sa surface extérieure ;
de telle sorte que la matière parfumée (4) reçoit uniquement de la chaleur reçue de la source de chaleur (2) par le premier élément (9) et transmise par conduction thermique par ledit premier élément (9) au troisième élément (11) puis par ledit troisième élément (11) au second élément (10).

2. Diffuseur de parfum selon la revendication 1, **caractérisé en ce que** les éléments (9, 10, 11) conducteurs de la chaleur sont agencés et dimensionnés de manière à transmettre par conduction thermique à la matière (4) un flux de chaleur suffisant pour chauffer ladite matière (4) à une température sensiblement constante comprise entre 40°C et 65°C environ.

3. Diffuseur de parfum selon la revendication 1 ou 2, **caractérisé en ce que** les trois éléments (9, 10, 11) conducteurs de la chaleur sont réalisés d'une seule pièce.

4. Diffuseur de parfum selon l'une des revendications 1 à 3, **caractérisé en ce qu'**il est réalisé principalement en métal bon conducteur de la chaleur et résistant à la température créée par la source (4), par exemple en aluminium ou en cuivre .

5. Diffuseur de parfum selon l'une des revendications 1 à 4, adapté à coopérer avec une source (2) de chaleur constituée par une flamme (7), **caractérisé en ce que** le premier élément (9) conducteur de la chaleur est une languette (9) inclinée par rapport à la direction de la flamme (7) et touchant ou non ladite flamme (7) à l'extrémité de celle-ci lors du fonctionnement du diffuseur (8).

6. Diffuseur de parfum selon la revendication 5, **caractérisé en ce que** l'inclinaison de la languette (9) par rapport à la direction de la flamme (7) est réglable.

7. Diffuseur de parfum selon l'une des revendications 5 ou 6, **caractérisé en ce qu'**il comporte des moyens pour modifier la distance entre la languette (9) et la flamme (7), par exemple un bilame.

8. Diffuseur de parfum selon l'une des revendications 1 à 7, **caractérisé en ce que** le second élément (10) conducteur de la chaleur est adapté à être noyé dans la matière (4), et **en ce que** le troisième élément conducteur (11) comporte un tronçon (12) adjacent au second élément conducteur (10) et adapté à être également noyé dans ladite matière.

9. Diffuseur de parfum selon la revendication 8, **caractérisé en ce que** le second élément (10) conducteur de la chaleur s'étend sensiblement sur toute la surface du fond (13) du récipient (3) contenant la matière (4).

10. Diffuseur de parfum selon la revendication 8 ou 9, **caractérisé en ce que** le second élément (10) conducteur de la chaleur repose sur le fond (13) du récipient (3) contenant la matière parfumée (4) par des entretoises (14) le maintenant à distance dudit fond (13).

11. Diffuseur de parfum selon l'une des revendications 5 à 10, **caractérisé en ce que** le troisième élément conducteur (11) présente, au moins dans le tronçon (12) dudit élément (11) qui est en contact avec la matière parfumée (4), une dimension transversale qui s'évase dans le sens vers le second élément (10).

12. Système (1) pour diffuser un parfum, comportant une source (2) de chaleur et un récipient (3) contenant une matière parfumée (4) adaptée à s'évaporer ou à se sublimer en diffusant un parfum, **caractérisé en ce qu'**il comporte un diffuseur de parfum (8) selon l'une quelconque des revendications 1 à 11.

## Claims

1. Perfume diffuser (8), disposed so as to co-operate with a heat source (2) and with means disposed so as to receive or constitute a container (3) holding a perfumed substance (4) disposed to evaporate or sublime diffusing a perfume, the diffuser (8) and these means being arranged in such a way as to transmit to said perfumed substance (4) a part of the heat generated by said source (2), **characterized in that** it comprises:
- a first heat-conducting element (9) disposed so as to receive a part of the heat generated by said heat source (2), and to transmit by thermal conduction the heat received;
- a second heat-conducting element (10) separate from the first element (9) and disposed so as to be in contact with said substance (4);
- a third heat-conducting element (11) linked to the first conducting element (9) and to the second conducting element (10) so as to communicate by thermal conduction to the second conducting element (10) the heat transmitted by the first conducting element (9),
- one or other or each of the first and third conducting elements (9, 11) if necessary being thermally insulated over at least one part of its outer surface.

2. Perfume diffuser according to Claim 1, **characterized in that** the heat-conducting elements (9, 10, 11) are disposed and proportioned so as to transmit by thermal conduction to the substance (4) a flow of heat sufficient to heat said substance (4) to a more or less constant temperature ranging between 40°C and 65°C approx.

3. Perfume diffuser according to Claim 1 or 2, **characterized in that** the three heat-conducting elements (9, 10, 11) are made in one piece.

4. Perfume diffuser according to one of Claims 1 to 3, **characterized in that** it is largely made of a metal which is a good conductor of heat and is resistant to the temperature generated by the source (4), for example aluminium or copper.

5. Perfume diffuser according to one of Claims 1 to 4, disposed so as to co-operate with a heat source (2) consisting of a flame (7), **characterized in that** the first heat-conducting element (9) is a tongue (9) inclined at an angle in relation to the direction of the flame (7) and touching or not touching the tip of said flame (7) during operation of the diffuser (8).

6. Perfume diffuser according to Claim 5, **characterized in that** the angle of the tongue (9) in relation to the direction of the flame (7) is adjustable.

7. Perfume diffuser according to one of Claims 5 or 6, **characterized in that** it includes means for altering the distance between the tongue (9) and the flame (7), for example a bimetallic strip.

8. Perfume diffuser according to one of Claims 1 to 7, **characterized in that** the second heat-conducting element (10) is disposed in such a way as to be submerged in the substance (4), and **in that** the third conducting element (11) includes a section (12) adjacent to the second conducting element (10) and disposed so as also to be submerged in said substance.

9. Perfume diffuser according to Claim 8, **characterized in that** the second heat-conducting element (10) extends more or less over the entire surface of the base (13) of the container (3) holding the substance (4).

10. Perfume diffuser according to Claim 8 or 9, **characterized in that** the second heat-conducting element (10) rests on the base (13) of the container (3) holding the perfumed substance (4) by way of struts (14) holding it at a distance from said base (13).

11. Perfume diffuser according to one of Claims 5 to 10, **characterized in that** the third conducting element (11) has, at least in the section (12) of said element (11) which is in contact with the perfumed substance (4), a crosswise dimension which widens out in the direction of the second element (10).

12. System (1) for diffusing a perfume, comprising a heat source (2) and a container (3) holding a perfumed substance (4) disposed so as to evaporate or sublime diffusing a perfume, **characterized in that** it comprises a perfume diffuser (8) according to any one of Claims 1 to 11.

## Patentansprüche

1. Duftdiffusor (8), der zum Zusammenwirken mit einer Wärmequelle (2) und mit Vorrichtungen geeignet ist, die geeignet sind, einen Behälter (3) aufzunehmen oder zu bilden, der einen Duftstoff (4) enthält, der geeignet ist, sich unter Diffusion eines Duftes zu verflüchtigen oder zu sublimieren, wobei der Diffusor (8) und die Vorrichtungen so angeordnet sind, daß ein Teil der von der Quelle (2) erzeugten Wärme an den Duftstoff (4) übertragen wird, **dadurch gekennzeichnet, daß** der Diffusor folgendes umfaßt:
- ein erstes wärmeleitendes Element (9), das geeignet ist, einen Teil der von der Wärmequelle (2) erzeugten Wärme aufzunehmen und die aufgenommene Wärme durch thermische Leitung zu übertragen;
- ein zweites wärmeleitendes Element (10), das vom ersten Element (9) getrennt ist und geeignet ist, mit dem Stoff (4) in Kontakt zu stehen;
- ein drittes wärmeleitendes Element (11), das mit dem ersten leitenden Element (9) und dem zweiten leitenden Element (10) so verbunden ist, daß es die durch das erste leitende Element (9) übertragene Wärme durch thermische Leitung an das zweite leitende Element (10) weitergibt; wobei
- das eine oder andere oder jedes der ersten und dritten leitenden Elemente (9, 11) gegebenenfalls an einem Teil zumindest seiner Außenfläche thermisch isoliert ist.

2. Duftdiffusor gemäß Anspruch 1, **dadurch gekennzeichnet, daß** die wärmeleitenden Elemente (9, 10, 11) so angeordnet und bemessen sind, daß auf den Stoff (4) durch thermische Leitung ein Wärmestrom übertragen wird, der ausreicht, den Stoff (4) auf eine im wesentlichen konstante Temperatur zwischen ungefähr 40°C und 65°C zu erwärmen.

3. Duftdiffusor gemäß Anspruch 1 oder 2, **dadurch gekennzeichnet, daß** die drei wärmeleitenden Elemente (9, 10, 11) aus einem einzigen Stück gestaltet sind.

4. Duftdiffusor gemäß einem der Ansprüche 1 bis 3, **dadurch gekennzeichnet, daß** er hauptsächlich aus einem gut wärmeleitenden Metall hergestellt ist, das gegenüber der von der Quelle (4) erzeugten Temperatur beständig ist, wie zum Beispiel aus Aluminium oder Kupfer.

5. Duftdiffusor gemäß einem der Ansprüche 1 bis 4, der zum Zusammenwirken mit einer von einer Flamme (7) gebildeten Wärmequelle (2) geeignet ist, **dadurch gekennzeichnet, daß** das erste wärmeleitende Element (9) eine Zunge (9) ist, die gegenüber der Richtung der Flamme (7) geneigt ist und die Flamme (7) beim Betrieb des Diffusors an deren Ende berührt oder nicht berührt.

6. Duftdiffusor gemäß Anspruch 5, **dadurch gekennzeichnet, daß** die Neigung der Zunge (9) gegenüber der Richtung der Flamme (7) einstellbar ist.

7. Duftdiffusor gemäß Anspruch 5 oder 6, **dadurch gekennzeichnet, daß** er Vorrichtungen zum Ändern des Abstands zwischen der Zunge (9) und der Flamme (7) umfaßt, wie zum Beispiel einen Bimetallstreifen.

8. Duftdiffusor gemäß einem der Ansprüche 1 bis 7, **dadurch gekennzeichnet, daß** das zweite wärmeleitende Element (10) geeignet ist, in den Stoff (4) eingetaucht zu sein und dadurch, daß das dritte leitende Element (11) einen Abschnitt (12) neben dem zweiten leitenden Element (10) umfaßt, der geeignet ist, ebenfalls in den Stoff eingetaucht zu sein.

9. Duftdiffusor gemäß Anspruch 8, **dadurch gekennzeichnet, daß** sich das zweite wärmeleitende Element (10) im wesentlichen über die gesamte Fläche des Bodens (13) des den Stoff (4) enthaltenden Behälters (3) erstreckt.

10. Duftdiffusor gemäß Anspruch 8 oder 9, **dadurch gekennzeichnet, daß** das zweite wärmeleitende Element (10) auf dem Boden (13) des den Duftstoff enthaltenden Behälters (3) mittels Stegen (14) lagert, die es im Abstand vom Boden (13) halten.

11. Duftdiffusor gemäß einem der Ansprüche 5 bis 10, **dadurch gekennzeichnet, daß** das dritte leitende Element (11) zumindest in dem Abschnitt (12) des Elements (11), der in Kontakt mit dem Duftstoff (4) steht, eine Querabmessung aufweist, die sich in Richtung zum zweiten Element (10) erweitert.

12. System (1) zur Diffusion eines Duftes, mit einer Wärmequelle (2) und einem Behälter (3), der einen Duftstoff (4) enthält, der geeignet ist, sich unter Diffusion von Duft (8) zu verflüchtigen oder zu sublimieren, **dadurch gekennzeichnet, daß** es einen Diffusor für Duft (8) gemäß einem der Ansprüche 1 bis 11 umfaßt.
